# EUROPÄISCHE PATENTSCHRIFT

(11) **EP 1 417 345 B1**
(45) Veröffentlichungstag und Bekanntmachung des Hinweises auf die Patenterteilung: **31.05.2006**
(21) Anmeldenummer: 02759869.7
(22) Anmeldetag: 19.08.2002
(51) Int. Cl.: C12Q 1/68

(54) **VERFAHREN UND SONDEN ZUR GENETISCHEN DIAGNOSE VON HERIDITÄRER HÄMOCHROMATOSE**
METHOD AND PROBES FOR THE GENETIC DIAGNOSIS OF HEREDITARY HAEMOCHROMATOSIS
PROCEDE ET SONDES PERMETTANT LE DIAGNOSTIC GENETIQUE D'UNE HEMOCHROMATOSE HEREDITAIRE

(30) Priorität: 17.08.2001 AT 20011295
(43) Veröffentlichungstag der Anmeldung: 12.05.2004
(73) Patentinhaber: Viennalab Labordiagnostika GmbH, 1110 Wien (AT)
(72) Erfinder: CAMASCHELLA, Clara, I-10128 Torino (IT); KURY, Friedrich, A-1180 Wien (AT); OBERKANINS, Christian, A-1060 Wien (AT)
(74) Vertreter: Grabherr, Claudia
(86) Internationale Anmeldenummer: PCT/AT2002/000250
(87) Internationale Veröffentlichungsnummer: WO 2003/016557

(56) Entgegenhaltungen:
- GIRELLI DOMENICO ET AL: "Clinical and pathologic findings in hemochromatosis type 3 due to a novel mutation in transferrin receptor 2 gene." GASTROENTEROLOGY, Bd. 122, Nr. 5, Mai 2002 (2002-05), Seiten 1295-1302, XP002224692 May, 2002 ISSN: 0016-5085
- DATABASE BIOSIS [Online] BIOSCIENCES INFORMATION SERVICE, PHILADELPHIA, PA, US; 16. November 2001 (2001-11-16) GIRELLI DOMENICO ET AL: "A new mutation in transferrin receptor 2 gene in hemochromatosis type 3." Database accession no. PREV200200129443 XP002224693 & BLOOD, Bd. 98, Nr. 11 Part 1, 16. November 2001 (2001-11-16), Seite 4a 43rd Annual Meeting of the American Society of Hematology, Part 1;Orlando, Florida, USA; December 07-11, 2001, November 16, 2001 ISSN: 0006-4971
- CAMASCHELLA C ET AL: "The gene TFR2 is mutated in a new type of haemochromatosis mapping to 7q22" NATURE GENETICS, NATURE AMERICA, NEW YORK, US, Bd. 25, 1. Mai 2000 (2000-05-01), Seiten 14-15, XP002185806 ISSN: 1061-4036

### SEQUENCE LISTING

<110> ViennaLab Labordiagnostika GmbH ViennaLab Labordiagnostika GmbH
<120> Verfahren und Sonde zur genetischen Diagnose von heriditärer Hämochrom atose
<130> 38504/VIENNALAB
<150> AT A 1295/2001
   <151> 2001-08-17
<160> 7
<170> PatentIn version 3.1
<210> 1
   <211> 2343
   <212> DNA
   <213> Homo sapiens
<400> 1
   atggagcggc tttggggtct attccagaga gcgcaacaac tgtccccaag atcctctcag      60
   accgtctacc agcgtgtgga aggcccccgg aaagggcacc tggaggagga agaggaagac     120
   ggggaggagg gggcggagac attggcccac ttctgcccca tggagctgag gggccctgag     180
   cccctgggct ctagacccag gcagccaaac ctcattccct gggcggcagc aggacggagg     240
   gctgccccct acctggtcct gacggccctg ctgatcttca ctggggcctt cctactgggc     300
   tacgtcgcct tccgagggtc ctgccaggcg tgcggagact ctgtgttggt ggtcagtgag     360
   gatgtcaact atgagcctga cctggatttc caccagggca gactctactg gagcgacctc     420
   caggccatgt tcctgcagtt cctgggggag gggcgcctgg aggacaccat caggcaaacc     480
   agccttcggg aacgggtggc aggctcggcc gggatggccg ctctgactca ggacattcgc     540
   gcggcgctct cccgccagaa gctggaccac gtgtggaccg acacgcacta cgtggggctg     600
   caattcccgg atccggctca ccccaacacc ctgcactggg tcgatgaggc cgggaaggtc     660
   ggagagcagc tgccgctgga ggaccctgac gtctactgcc cctacagcgc catcggcaac     720
   gtcacgggag agctggtgta cgcccactac gggcggcccg aagacctgca ggacctgcgg     780
   gccaggggcg tggatccagt gggccgcctg ctgctggtgc gcgtgggggt gatcagcttc     840
   gcccagaagg tgaccaatgc tcaggacttc ggggctcaag gagtgctcat atacccagag     900
   ccagcggact tctcccagga cccacccaag ccaagcctgt ccagccagca ggcagtgtat     960
   ggacatgtgc acctgggaac tggagacccc tacacacctg gcttcccttc cttcaatcaa    1020
   acccagaagc tcaaaggccc tgtggccccc caagaatggc aggggagcct cctaggctcc    1080
   ccttatcacc tgggccccgg gccacgactg cggctagtgg tcaacaatca caggacctcc    1140
   acccccatca acaacatctt cggctgcatc gaaggccgct cagagccaga tcactacgtt    1200
   gtcatcgggg cccagaggga tgcatggggc ccaggagcag ctaaatccgc tgtggggacg   1260
   gctatactcc tggagctggt gcggaccttt tcctccatgg tgagcaacgg cttccggccc   1320
   cgcagaagtc tcctcttcat cagctgggac ggtggtgact ttggaagcgt gggctccacg   1380
   gagtggctag agggctacct cagcgtgctg cacctcaaag ccgtagtgta cgtgagcctg   1440
   gacaacgcag tgctggggga tgacaagttt catgccaaga ccagccccct tctgacaagt   1500
   ctcattgaga gtgtcctgaa gcaggtggat tctcccaacc acagtgggca gactctctat   1560
   gaacaggtgg tgttcaccaa tcccagctgg gatgctgagg tgatccggcc cctacccatg   1620
   gacagcagtg cctattcctt cacggccttt gtgggagtcc ctgccgtcga gttctccttt   1680
   atggaggacg accaggccta cccattcctg cacacaaagg aggacactta tgagaacctg   1740
   cataaggtgc tgcaaggccg cctgcccgcc gtggcccagg ccgtggccca gctcgcaggg   1800
   cagctcctca tccggctcag ccacgatcgc ctgctgcccc tcgacttcgg ccgctacggg   1860
   gacgtcgtcc tcaggcacat cgggaacctc aacgagttct ctggggacct caaggcccgc   1920
   gggctgaccc tgcagtgggt gtactcggcg cggggggact acatccgggc ggcggaaaag   1980
   ctgcggcagg agatctacag ctcggaggag agagacgagc gactgacacg catgtacaac   2040
   gtgcgcataa tgcggatccc cctctctgcg caggtggagt tctacttcct ttcccagtac   2100
   gtgtcgccag ccgactcccc gttccgccac atcttcatgg gccgtggaga ccacacgctg   2160
   ggcgccctgc tggaccacct gcggctgctg cgctccaaca gctccgggac ccccggggcc   2220
   acctcctcca ctggcttcca ggagagccgt ttccggcgtc agctagccct gctcacctgg   2280
   acgctgcaag gggcagccaa tgcgcttagc ggggatgtct ggaacattga taacaacttc   2340
   tga                                                                 2343
<210> 2
   <211> 45
   <212> DNA
   <213> Homo sapiens
<400> 2
   cccgccgtgg cccaggccft ggcccagctc gcagggcagc tcctc 45
<210> 3
   <211> 33
   <212> DNA
   <213> Homo sapiens
<400> 3
   cccgccgtgg cccagctcgc agggcagctc ctc 33
<210> 4
   <211> 15
   <212> PRT
   <213> Homo sapiens
<400> 4
   Pro Ala Val Ala Gln Ala Val Ala Gln Leu Ala Gly Gln Leu Leu
   1               5                   10                  15
<210> 5
   <211> 11
   <212> PRT
   <213> Homo sapiens
<400> 5
   Pro Ala Val Ala Gln Leu Ala Gly Gln Leu Leu
   1               5                   10
<210> 6
   <211> 23
   <212> DNA
   <213> Homo sapiens
<400> 6
   cccagcgtcc accctgtcct ggc 23
<210> 7
   <211> 20
   <212> DNA
   <213> Homo sapiens
<400> 7
   ctggattgcc agagaggacc 20

## Beschreibung

Die Erfindung betrifft Verfahren zur genetischen Diagnose von heriditärer Hämochromatose (HH) sowie Sonden zur genetischen Diagnose von heriditärer Hämochromatose.

Hämochromatose ist durch eine Störung im Eisenstoffwechsel des menschlichen Körpers gekennzeichnet, die zu einer Eisenüberladung führt. Das überschüssige Eisen lagert sich in einer Vielzahl von Organen ab und verursacht irreversible Schäden und daraus resultierende Krankheiten. Da die klinischen Symptome der Hämochromatose oft erst im Alter von 40, 50 oder mehr Jahren auftreten, dann aber schon irreversible Organschäden vorliegen können, gibt es viele Befürworter einer präsymptomatischen Diagnostik. Umso mehr, als durch regelmäßigen Aderlass das übermäßig aufgenommene Eisen einfach entfernt werden kann.

Im Zusammenhang mit Hämochromatose wurden bisher zahlreiche Mutationen im HFE-Gen (Chromosom 6p, HH Typ 1) gefunden (Feder et al., A novel MHC class I-like gene is mutated in patients with hereditary haemochromatosis, Nat.Genet. 1996, 13:399-408; Villiers et al., Spectrum of mutations in the HFE gene implicated in haemochromatosis and porphyria, Hum.Mol.Genet. 1999, 8:1517-22). Der Nachweis dieser Mutationen in biologischen Proben kann für die genetische Diagnose von Hämochromatose herangezogen werden.

Eine bekannte Hämochromatoseerkrankung, die nicht auf Mutationen im HFE-gen beruht, ist die juvenile Hämochromatose (HH Typ 2). Ihre Ursache wurde am Chromosom 1 q lokalisiert.

Vor kurzem wurden auch Mutationen im TFR2-Gen (Chromosom 7q22, HH Typ 3) gefunden, die in Zusammenhang mit Hämochromatose stehen. Sie sind gemeinsam mit weiteren neuen Mutationen im HFE-gen in der internationalen Anmeldung PCT/EP01/04835 (WO 01/83812) beschrieben.

Es ist Aufgabe der vorliegenden Erfindung weitere Möglichkeiten zu finden genetische Ursachen für Hämochromatose zu diagnostizieren, um bestehende Untersuchungsverfahren, die auf bisher bekannte genetische Ursachen untersuchen, zu ergänzen und ein noch sichereres Untersuchungsergebnis zu erzielen.

Die Aufgabe wird dadurch gelöst, daß eine biologische Probe auf Vorliegen der Nukleotidfolge
5' cccgccgtggcccagctcgcagggcagctcctc 3' (Sequenz Nr.3) anstelle der Nukleotidfolge 5' cccgccgtggcccaggccgtggcccagctcgcagggcagctcctc 3' (Sequenz Nr.2) aufgrund einer 12 Nukleotide langen Deletion im Exon 16 der TFR2 cDNA-Sequenz untersucht wird.

Insbesondere wird erfindungsgemäss die Probe auf Vorliegen einer Deletion der Nukleotide 1780-1791 im Exon 16 der TFR2-α cDNA-Sequenz untersucht. Bezüglich der TFR2-α cDNA Sequenz sei auf die GenBank Zugangsnummer NM_003227.1 und das beiliegende Sequenzprotokoll (Sequenz Nr. 1) verwiesen.

Diese Mutation wurde bei drei hämochromatosekranken Geschwistern gefunden, die keine der bekannten HFE-Mutationen zeigten. Die Mutation ist eine 12 Nukleotide lange Deletion einer wiederholten Sequenz in Exon 16 des TFR2-α-gens und wurde durch direktes Sequenzieren gefunden.

Man fand an Stelle der normalen Sequenz
5' cccgccgtggcccaggccgtggcccagctcgcagggcagctcctc 3' (Sequenz Nr.2)
5' cccgccgtggcccagctcgcagggcagctcctc 3' (Sequenz Nr.3)
als mutierte Sequenz.

Sie lag im homozygoten Zustand vor und wurde assoziiert mit dem Phänotyp in der untersuchten Familie vererbt. Sie war in 100 normale Chromosomen nicht vorhanden.

Als biologische Probe kann dabei Blut, Gewebs-Biopsie, Mundhöhlenabstrich, Fruchtwasser etc. verwendet werden, wobei die biologische Probe, je nach Auswahl des an sich bekannten Untersuchungsverfahrens, ebenfalls bekannten Vorbehandlungen unterworfen wird.

Ebenso wird die Aufgabe dadurch gelöst, daß eine biologische Probe auf Vorliegen von Nukleinsäuren untersucht wird, die für ein TFR2-genprodukt kodieren mit einer Aminosäurenfolge Pro Ala Val Ala Gln **Leu Ala Gly Gln** Leu Leu (Sequenz Nr. 5) anstelle der Aminosäurenfolge Pro Ala Val Ala Gin **Ala Val Ala Gln** Leu Ala Gly Gln Leu Leu (Sequenz Nr. 4) oder auf Vorliegen eines TFR2-genproduktes mit einer Aminosäurenfolge
Pro Ala Val Ala Gln **Leu Ala Gly Gln** Leu Leu (Sequenz Nr. 5) anstelle der Aminosäurenfolge Pro Ala Val Ala Gln **Ala Val Ala Gln** Leu Ala Gly Gln Leu Leu (Sequenz Nr. 4) untersucht wird.

Insbesondere wird die Probe auf Vorliegen von Nukleinsäuren untersucht, die für ein TFR2-α-genprodukt kodieren mit einer Aminosäurenfolge Leucin-Alanin-Glycin-Glutamin für die Aminosäuren 594-597 oder auf Vorliegen eines TFR2-α-genproduktes mit einer Aminosäurenfolge Leucin-Alanin-Glycin-Glutamin für die Aminosäuren 594-597.

Man findet an Stelle der normalen Aminosäurenfolge
Pro Ala Val Ala Gln **Ala Val Ala Gln** Leu Ala Gly Gln Leu Leu (Sequenz Nr. 4)
Pro Ala Val Ala Gln **Leu Ala Gly Gln** Leu Leu (Sequenz Nr. 5).

Wird die biologische Probe auch auf Vorliegen von Nukleinsäuren untersucht, deren TFR2-Gen die genannten Mutation oder die zuletzt genannte Aminosäurefolge nicht aufweist, lässt sich nachweisen ob die Mutation homozygot oder heterozygot vorliegt.

Die Untersuchung kann in an sich bekannter Weise durch Sequenzierung der aus der biologischen Probe erhaltenen Nukleinsäure durchgeführt werden.

Oder aber die Nukleinsäuren der biologischen Probe werden mit mindestens einer Sonde in Kontakt gebracht, die fähig ist mit Nukleinsäuren einer biologischen Probe in einem Bereich, der die Nukleotidfolge 5' cccgccgtggcccagctcgcagggcagctcctc 3' (Sequenz Nr.3) im Exon 16 der TFR2 cDNA-Sequenz enthält zu hybridisieren und es wird überprüft, ob entsprechende Hybridisierungsprodukte vorliegen.

Insbesondere werden die Nukleinsäuren der biologischen Probe mit mindestens einer Sonde in Kontakt gebracht, die fähig ist mit einem Bereich dieser Nukleinsäuren, der einem Bereich im Exon 16 der TFR2-α cDNA-Sequenz im Bereich der Nukleotide 1780-1791 entspricht, zu hybridisieren wenn eine Deletion der Nukleotide 1780-1791 vorliegt und es wird überprüft, ob entsprechende Hybridisierungsprodukte vorliegen.

Werden die Nukleinsäuren der biologischen Probe mit mindestens einer Sonde in Kontakt gebracht, die fähig ist mit einem Bereich dieser Nukleinsäuren, der die Nukleotidfolge 5' cccgccgtggcccaggccgtggcccagctcgcagggcagctcctc 3' (Sequenz Nr.2) enthält zu hybridisieren bzw. mit einem Bereich dieser Nukleinsäuren, der einem Bereich im Exon 16 der TFR2-α cDNA-Sequenz im Bereich der Nukleotide 1780-1791 entspricht, zu hybridisieren und wird überprüft ob entsprechende Hybridisierungsprodukte vorliegen, kann festgestellt werden ob die Mutation homozygot oder heterozygot vorliegt.

Derzeit werden eine Vielzahl von Verfahren zur Analyse von Mutationen verwendet, unter anderem RFLP (restriction fragment length polymorphism), PCR-SSP (PCR with sequencespecific primers), Allelespezifische Oligonukleotidhybridisierung, SSCP (single-strand conformation polymorphism, DGGE (denaturing gradient gel electrophoresis), OLA (oligonucleotide litigation assay), Echtzeit-Fluoreszenz-PCR und DNA Sequenzierung. Wenn RNA untersucht wird, kann das z.B. mittels sog. NASBA™ ("Nucleic acid sequence-based amplification", Nature (1991) Mar 7; 350(6313):91-2, Compton J.; entwickelt von Cangene Corporation , Mississauga, Ontario, Canada) erfolgen, oder es kann auch RNA mittels reverser Transkription in cDNA umgeschrieben werden, dann mit PCR amplifiziert und anschließend charakterisiert werden. All diese bekannten Analyseverfahren können für das erfindungsgemäße Diagnoseverfahren herangezogen werden.

Die erfindungsgemäße Sonde zur Diagnose von Hämochromatose ist fähig mit Nukleinsäuren einer biologischen Probe in einem Bereich, der die Nukleotidfolge
5' cccgccgtggcccagctcgcagggcagctcctc 3' (Sequenz Nr.3) im Exon 16 der TFR2 cDNA-Sequenz enthält zu hybridisieren.
Eine erfindungsgemässe Sonde ist fähig mit Nukleinsäuren einer biologischen Probe in einem Bereich zu hybridisieren, der einem Bereich im Exon 16 der TFR2-α cDNA-Sequenz im Bereich der Nukleotide 1780-1791 entspricht, wenn eine Deletion der Nukleotide 1780-1791 vorliegt.

Im Folgenden wird über die Familie, bei der die neue Mutation gefunden wurde und die diesbezüglichen Untersuchungen berichtet.

Bei einem 32 Jahre altem Mann sollte eine bereits früher entdeckte Erkrankung mit Eisenüberladung neuerlich beurteilt werden. Die klinische Geschichte zeigte weder Alkoholkonsum, noch Bluttransfusionen oder außergewöhnliche orale Eiseneinnahme.

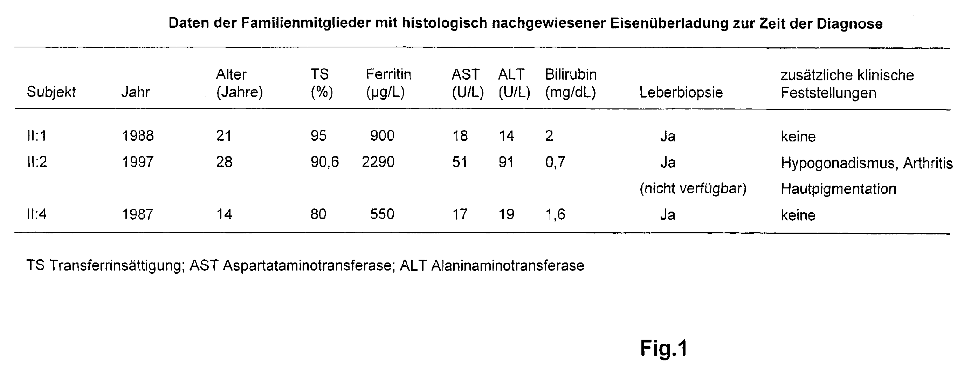

Im Alter von 16 Jahren wurden bei biochemischen Untersuchungen auf Sklerenikterus erhöhte Serumspiegel von Bilirubin (2,8 mg/dL, vorwiegend unverbunden) und Eisen (230 µg/dL) gefunden. Eine ausgedehnte hämatologische Beurteilung schloss das Vorhandensein irgendeiner Form von kompensierter Eisen speichernden Anämie aus und es wurde ein Gilbert Syndrom auf klinischer Basis diagnostiziert. Zu der Zeit war der Serumferritinspiegel 520 µg/L und die Transferrinsättigung war 76%. Im Alter von 21 Jahren wurde aufgrund der ständigen Abnormalitäten der biochemischen Parameter (Fig.1) eine Leberbiopsie durchgeführt. Obwohl eine Hämochromatosediagnose erstellt wurde, wurde nicht mit einer Phlebotomietherapie begonnen, und der Patient wurde mit intravenösem Deferoxamin (1 g, 3 Tage die Woche, durch Infusionspumpe) für 4 Jahre behandelt. Am Ende der Behandlung war der Serumferritinspiegel bei 553 µg/L. In den folgenden Jahren wurde keine Behandlung durchgeführt obwohl der Serumferritinspiegel ständig >1000 µg/L war.

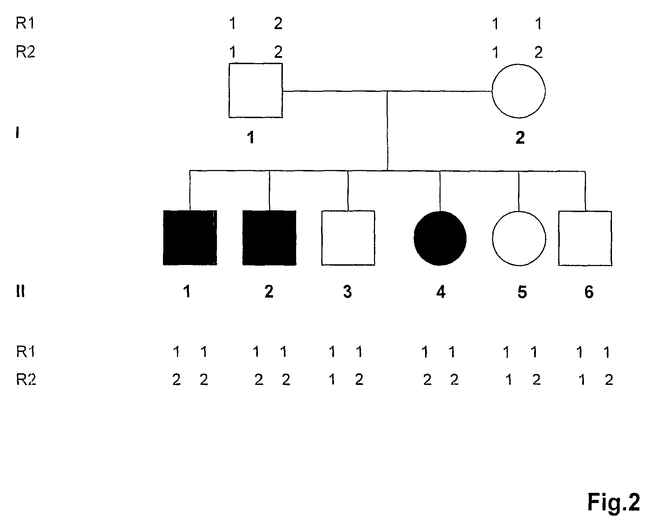

Beide Elternteile stammten seit mehreren Generationen aus Norditalien und waren nicht symptomatisch. Die Durchsicht der klinischen Unterlagen der Familienmitglieder zeigte, dass zwei Geschwister vorangegangene Diagnosen bezüglich Eisenüberladung hatten. Der Stammbaum der Familie ist in Fig.2 wiedergegeben. Die erkrankten Familienmitglieder sind durch gefüllte Symbole dargestellt. Eine intrafamiliäre Segregation von zwei Sequenzwiederholungen (R1 und R2) im TFR2-gen, die identische Haplotypen aufweisen, wurde bei den erkrankten Geschwistern festgestellt.

EDTA Blutproben wurden für genetische Untersuchungen entnommen. DNA wurde durch herkömmlich Phenol-Chloroform-Extraktion (Sambrook J., Frisch E., Maniatis T, Molecular cloning: a laboratory manual., Cold Spring Habor, NY: Cold Spring Habor Laboratory Press, 1989) gewonnen. Unter Verwendung von 12,5 pmol Primer und 0,5 U Taq Polymerase wurde in einem Thermozyküergerät (perker Elmer, Shelto, CT) für 30 bis 35 Zyklen PCR (polymerase chain reaction) in einer Gesamtmenge von 50µL durchgeführt.

C282Y und H63D Mutationen in HFE wurden anhand von amplifizierter genomischer DNA unter Verwendung von PCR-basierten Tests und Verdau mit Restriktionsenzymen Rsa I und Mbo I (New England Biolabs, Berkeley, MA) wie beschrieben (Carella M, D'Ambrosio L, Totaro A, Grifa A, Valentino MA, Piperno A, Girelli D, Roetto A, Franco B, Gasparini P, Camaschella C. Mutation analysis of HLA-H gene in Italian hemochromatosis patients. Am J Hum Genet 1997;60:828-32) untersucht.

An HH Typ 2 und Typ 3 loci wurde jeweils eine Linkage Analyse durch intrafamiliäre Segregation von Mikrosatellitenmarkerallele der Chromosomen 1q und 7q22 wie vorbeschrieben durchgeführt. Mikrosatellitenmarker für das linken von Chromosom 1 q waren D1S2344, D1S442, D1S1156, GATA13C08 und D1S498 (Roetto A., Totaro A., Cazzola M, Cicilano M., Bosio S., D'Ascola G., Carella M., Zelante L., Kelly A.L., Cox T.M., Gasparini P., Camaschella C., The juvenile hemochromatosis locus maps to chromosome 1q., Am J Hum Genet 1999, 64:1388-1393). Mikrosatellitenmarker für Chromosom 7q waren D7S651, D7S2498, D7S662, D7S477, D7s1588 und 2 TFR2 Intragenwiederholungen (R1 und R2) (Camaschella C., Roetto A., Cali A., De Gobbi M., Garozzo G., Carella M., Majorano N., Totaro A., Gasparini P., The gene encoding transferrin receptor 2 is mutated in a new type of hemochromatosis mapping to 7q22., Nat Genet 2000;25:14-15;; Roetto A., Totaro A., Piperno A, Piga A., Longo F., Garozzo G., Cali A., De Gobbi M., Gasparini P., Camaschella C., New mutations inactivating transferrin receptor 2 in hemochromatosis type 3. , Blood 2001; 97:2555-2560). Primer wurden entsprechend zu Datenbanksequenzen hergestellt.

Direktes Sequenzieren von gereinigten PCR-produkten wurde unter Verwendung von ThermoSequenase Cy 5.5 Sequencing Kit (Amersham-Pharmacia, Uppsala, Schweden) und einem Seq 4 x 4 Apparatus (Amersham Pharmacia Biotech, Uppsala, Schweden) durchgeführt.

Acrylamidgelelektrophorese wurde in einem genePhor-Elektrophoresesystem (Amersham Pharmacia Biotech) mit 6%-Fertig-Acrylamidgelen durchgeführt. Eine Analyse der (TA)₇ Promotorinsertion in Bilirubinuridindphosphat-glucuronosyltransferase (UGT)1-Gen wurde wie beschrieben durchgeführt (Bosma P.J., ChowdhuryJ.R. , BakkerC., Gantla S., de Boer A., Oostra B.A., Lindhout D., Tytgat G.N.J., Jansen P.L.M., Elferink R.P.J.O., Chowdhury N.R., The genetic basis of the reduced expression of bilirubin UDP-glucuronosyltransferase 1 in Gilbert's syndrome, N.Engl.J.Med 1995 , 333:1171-1175).

Kontrollen waren 100 gesunde Blutspender aus der selben geografischen Region wie die Familie.

### Ergebnisse

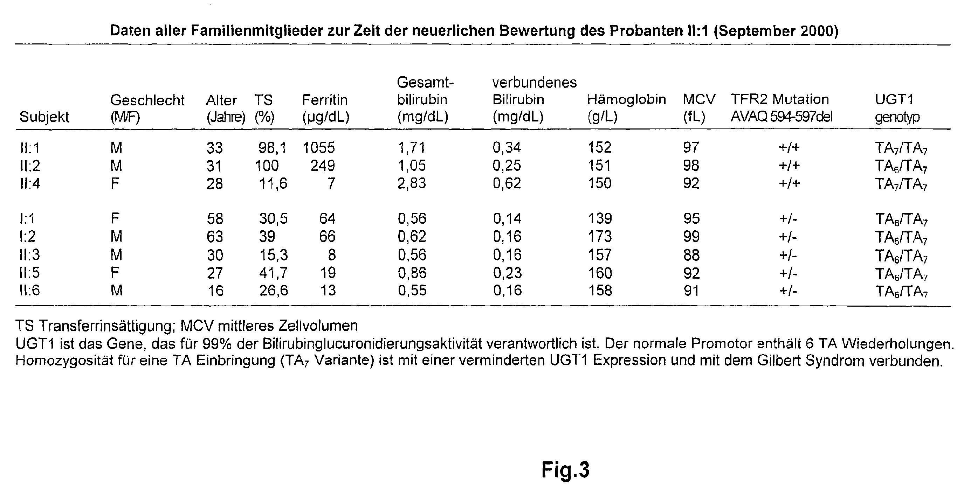

Die erste Leberbiopsieprobe der Testperson wurde wiedergefunden und nochmals begutachtet. Die histopathologische Bewertung zeigte normalen lappenförmigen Aufbau, keine signifikante Fibrose und hepatozelluläre Hemosiderinablagerungen vom Grad III nach Scheuer et al. (Scheuer P.J., Williams R., Muir A.R., Hepatic pathology in relatives of patients with hemochromatosis, J.Pathol.Bacteriol 1962;84:53-64). Die biochemischen Daten der Testperson zum Zeitpunkt der Wiederbewertung sind in Fig.3 wiedergegeben. Bei der körperlichen Untersuchung war der Leberrand etwas empfindlich und 3 cm unter dem rechten Rippenrand tastbar. Es war keine Hautpigmentation erkennbar. Es gab weder klinische noch biochemische Anzeichen von entweder diabetes mellitus oder Hypogonadismus. Elektrokardiographie und Echokardiographie waren normal. Da der Patient für einen langen Zeitraum keine geeignete Therapie erhalten hatte wurde eine neue Biopsie zur Erhebung des Status der Lebererkrankung durchgeführt. Die histopathologische Bewertung zeigte einen normalen lappenförmigen Aufbau, nur einen leichten Grad von Fibrose, und hepatozelluläre Hemosiderinablagerungen vom Grad IV nach Scheuer et al.. Die Eisenkonzentration in der Leber war 358 µmol/g Trockengewicht, und der Eisenindex in der Leber (Verhältnis von Eisenkonzentration [µmol/g Trockengewicht] in der Leber zu Alter [Jahren]) war 11,2. Danach wurde eine Phlebotomietherapie begonnen und es wurden nach Entfernen von 8,75g Eisen ein Serumferritinspiegel (46 µg/L) erreicht, der unter den Normalwerten lag, wohingegen die Transferrinsättigung erhöht (100%) blieb.

Die Daten der beiden erkrankten Geschwister zum Zeitpunkt der Diagnose der Eisenüberladung sind in Fig.1 wiedergegeben. Der 31 Jahre alte Bruder (11:2) hatte Arthritis und Hypogonadismus. Er konsumierte ca. 40g Alkohol täglich. Bei diesem Probanten wurde nach Entfernen von 12,25 g Eisen ein normaler Serumferritinspiegel (120µg/L) erreicht. Währendessen blieb die Transferrinsättigung hoch (100%).

Die 28 Jahre alte Schwester (II:4) wurde mit 14 Jahren aufgrund eines Sklerenikterus zum ersten Mal untersucht. Zu dieser Zeit wurde eine Leberbiopsie gemacht, die wiedergefunden wurde und nochmals begutachtet wurde. Die klinische Geschichte zeigte, dass die Patientin einige Monate nach der Leberbiopsie eine Essstörung mit fortschreitetendem, grossem Gewichtsverlust entwickelte. Anorexia nervosa wurde diagnostiziert und sie wurde bis zum zwanzigsten Lebensjahr mit Psychotherapie behandelt, in welchem sich die Essstörung wesentlich besserte. Als ihr Gewicht stabil über 50 kg betrug wurde sie als regelmäßiger Blutspender angeworben. Nach acht Blutspenden (jeweils 350mL) verminderte sich der Hämoglobinspiegel auf 12g/dL. Im selben Zeitraum unterzog sie sich einer Esophagogastro-duodenoskopie aufgrund von anhaltenden dyspeptischen Symptomen. Die Endoskopie bestätigte das Vorhandensein einer chronischen Gastritis im präpylorischen Magenabschnitt aufgrund einer Heliobacter pylori Infektion. Die biochemischen Daten der Patientin zur Zeit der Wiederbewertung, sowie jene aller anderen Familienmitglieder sind in Fig.3 wiedergegeben.

### Molekulare Studien

Keine der bekannten HFE-Mutationen wurde in irgendeinem Familienmitglied gefunden. Juvenile Hämochromatose wurde ausgeschlossen da die erkrankten Geschwister verschiedene 1q Haplotypen in der HH Typ2 kritischen Region hatten.

Intrafamiliäre Segregation von Markerallelen des Chromosoms 7q passten mit der Verbindung zum HH Typ3 locus zusammen; die drei erkrankten Geschwister waren haplotypidentisch, und ihre 7q Haplotypen waren von jenen der nicht erkrankten Geschwister verschieden. Die Ergebnisse betreffend der Sequenzwiederholungen R1 und R2 im TFR2-gen sind in Fig.2 wiedergegeben.

Eine Mutationsanalyse der gesamten TFR2-α kodierenden Sequenz (18 Exons) und der Exon/Intron-grenzen der genomischen DNA der Patienten II:2 und II:4 ergab eine einzige idente homozygote Mutation in beiden Subjekten. Die Mutation war eine 12 Nukleotide lange Deletion in einer 12 Nukleotide langen Wiederholung in Exon 16 an der Position 1780 - 1791. Diese Deletion verursachte ein 4-Aminosäuren (AVAQ) lange Deletion an der Position 594 - 597 im Protein.

Da ein um 12 Nucleotide kürzeres PCR-fragment nach Amplifikation von Exon 16 erhalten wurde, wurde die Segregation der AVAQ-Mutation durch Akrylamidgelelektrophorese analysiert. Diese Analyse zeigte, dass in der Familie die Segregation der Deletion mit der Krankheit erfolgt; sie war im homozygoten Zustand in den Probanten 11:1, 11:2 und II:4 und im heterozygoten Zustand in allen anderen Familienmitgliedern vorhanden (Fig.3). Die Deletion wurde unter 100 gesunden Kontrollen nicht gefunden.

Aufgrund der ständig erhöhten Bilirubinspiegel bei manchen Familienmitgliedern wurde die (TA)-Repeat-Insertion in den UGT1-gen-promoter analysiert. Ein (TA)₇ /(TA)₇ Muster zeigte sich bei Familienmitgliedern mit Hyperbilirubinemia (II:1 und II:4) im Einklang mit der Diagnose eines Gilbert Syndroms.

Das folgende Beispiel beschreibt mögliche Ausführungsformen eines erfindungsgemäßen Verfahrens zur genetischen Diagnose von Hämochromatose.

### DNA Isolation und Amplifikation:

DNA wurde aus antikoaguliertem Blut unter Verwendung herkömmlicher Extraktionsverfahren ("A simple salting out procedure for extracting DNA from human nucleated cells" Miller et al., 1988) oder kommerziell erhältlicher Reagenzien (GenXtract DNA extraction system, ViennaLab, Wien, AT) isoliert. Die Exon 16- Sequenzen des TFR2-α-Gens wurde in einer PCR-Reaktion unter Verwendung der Primer 5'-cccagcgtccaccctgtcctggc-3' (Sequenz Nr. 6) und 5'-ctggattgccagagaggacc-3' (Sequenz Nr.7) amplifiziert. Jeder Primer wurde mit einer Endkonzentration von 25 pM verwendet.

Ein Thermozyklierprogramm mit 30 Zyklen (94°C für 15s, 58°C für 30s, und 72°C für 30s) wurde durchgeführt mit dem GeneAmp PCR System 2400 (PE Biosystems, Foster City, CA).

### Sequenzieren:

Die PCR Produkte wurde mit "Centricon-100"Säulen entsprechend den Empfehlungen des Herstellers gereinigt. Die amplifizierte und gereinigte DNA wurde mit UV-Spektrophotometrie quantifiziert und anschließend sequenziert unter strikter Einhaltung des Protokolls aus "BigDye Terminator Cycle Sequencing Ready Reaction Kit - with AmpliTaq DNA Polymerase, FS" (PE Applied Biosystems) für die ABI Prism instrumentation (PE Applied Biosystems).

## Patentansprüche

1. Verfahren zur Diagnose von Hämochromatose, **dadurch gekennzeichnet, daß** eine biologische Probe auf Vorliegen der Nukleotidfolge
5' cccgccgtggcccagctcgcagggcagctcctc 3' (Sequenz Nr.3) anstelle der Nukleotidfolge 5' cccgccgtggcccaggccgtggcccagctcgcagggcagctcctc 3' (Sequenz Nr.2) aufgrund einer 12 Nukleotide langen Deletion im Exon 16 der TFR2 cDNA-Sequenz untersucht wird.

2. Verfahren nach Anspruch 1, **dadurch gekennzeichnet, dass** eine biologische Probe auf Vorliegen einer Deletion der Nukleotide 1780-1791 im Exon 16 der TFR2-α cDNA-Sequenz (Sequenz Nr. 1) untersucht wird.

3. Verfahren zur Diagnose von Hämochromatose, **dadurch gekennzeichnet, daß** eine biologische Probe auf Vorliegen von Nukleinsäuren untersucht wird, die für ein TFR2-produkt kodieren mit einer Aminosäurenfolge
Pro Ala Val Ala Gln **Leu Ala Gly Gln** Leu Leu (Sequenz Nr. 5) anstelle der Aminosäurenfolge Pro Ala Val Ala Gln **Ala Val Ala Gln** Leu Ala Gly Gln Leu Leu (Sequenz Nr. 4)
oder auf Vorliegen eines TFR2-α-genproduktes mit einer Aminosäurenfolge
Pro Ala Val Ala Gln **Leu Ala Gly Gln** Leu Leu (Sequenz Nr. 5) anstelle der Aminosäurenfolge Pro Ala Val Ala Gln **Ala Val Ala Gln** Leu Ala Gly Gln Leu Leu (Sequenz Nr. 4).

4. Verfahren nach Anspruch 3, **dadurch gekennzeichnet, dass** eine biologische Probe auf Vorliegen von Nukleinsäuren untersucht wird, die für ein TFR2-α-produkt kodieren mit einer Aminosäurenfolge Leucin-Alanin-Glycin-Glutamin für die Aminosäuren 594-597 oder auf Vorliegen eines TFR2-α-genproduktes mit einer Aminosäurenfolge Leucin-Alanin-Glycin-Glutamin für die Aminosäuren 594-597.

5. Verfahren nach einem der vorhergehenden Ansprüche, **dadurch gekennzeichnet, daß** die biologische Probe auch auf Vorliegen von Nukleinsäuren untersucht wird, deren TFR2-Gen die genannte Mutation oder die genannte geänderte Aminosäurefolge nicht aufweist.

6. Verfahren nach einem der vorhergehenden Ansprüche, **dadurch gekennzeichnet, daß** die Untersuchung in an sich bekannter Weise durch Sequenzierung der aus der biologischen Probe erhaltenen Nukleinsäure durchgeführt wird.

7. Verfahren nach Anspruch 1, **dadurch gekennzeichnet, daß** Nukleinsäuren der biologischen Probe mit mindestens einer Sonde in Kontakt gebracht werden, die fähig ist mit einem Bereich dieser Nukleinsäuren, der die Nukleotidfolge 5' cccgccgtggcccagctcgcagggcagctcctc 3' (Sequenz Nr.3) enthält zu hybridisieren und daß überprüft wird ob entsprechende Hybridisierungsprodukte vorliegen.

8. Verfahren nach Anspruch 2 und 7, **dadurch gekennzeichnet, daß** Nukleinsäuren der biologischen Probe mit mindestens einer Sonde in Kontakt gebracht werden, die fähig ist mit einem Bereich dieser Nukleinsäuren, der einem Bereich im Exon 16 der TFR2-α cDNA-Sequenz im Bereich der Nukleotide 1780-1791 entspricht, zu hybridisieren wenn eine Deletion der Nukleotide 1780-1791 vorliegt und daß überprüft wird ob entsprechende Hybridisierungsprodukte vorliegen.

9. Verfahren nach Anspruch 7, **dadurch gekennzeichnet, daß** Nukleinsäuren der biologischen Probe mit mindestens einer Sonde in Kontakt gebracht werden, die fähig ist mit einem Bereich dieser Nukleinsäuren, der die Nukleotidfolge
5' cccgccgtggcccaggccgtggcccagctcgcagggcagctcctc 3' (Sequenz Nr.2) enthält zu hybridisieren und daß überprüft wird ob entsprechende Hybridisierungsprodukte vorliegen.

10. Verfahren nach Anspruch 8, **dadurch gekennzeichnet, daß** Nukleinsäuren der biologischen Probe mit mindestens einer Sonde in Kontakt gebracht werden, die fähig ist mit einem Bereich dieser Nukleinsäuren, der einem Bereich im Exon 16 der TFR2-α cDNA-Sequenz im Bereich der Nukleotide 1780-1791 entspricht, zu hybridisieren wenn keine Mutation im Bereich der Nukleotide 1780-1791 vorliegt und daß überprüft wird ob entsprechende Hybridisierungsprodukte vorliegen.

11. Sonde zur Diagnose von Hämochromatose, **dadurch gekennzeichnet, daß** die Sonde fähig ist mit Nukleinsäuren einer biologischen Probe in einem Bereich, der die Nukleotidfolge 5' cccgccgtggcccagctcgcagggcagctcctc 3' (Sequenz Nr.3) im Exon 16 der TFR2 cDNA-Sequenz enthält zu hybridisieren.

12. Sonde nach Anspruch 11, **dadurch gekennzeichnet, daß** die Sonde fähig ist mit Nukleinsäuren einer biologischen Probe in einem Bereich, der einem Bereich im Exon 16 der TFR2-α cDNA-Sequenz im Bereich der Nukleotide 1780-1791 entspricht, zu hybridisieren wenn eine Deletion der Nukleotide 1780-1791 vorliegt.

## Claims

1. Method of diagnosing hemochromatosis, **characterized in that** a biological sample is analyzed for the presence of the nucleotide sequence 5' cccgccgtggcccagctcgcagggcagctcctc 3' (Sequence No. 3) instead of nucleotide sequence 5' cccgccgtggcccaggccgtggcccagctcgcagggcagctcctc 3' (Sequence No. 2) as a result of a 12 nucleotide long deletion in Exon 16 of the TFR2 cDNA sequence.

2. Method according to Claim 1, **characterized in that** a biological sample is analyzed for the presence of a deletion of the nucleotides 1780-1791 in Exon 16 of the TFR2-α cDNA sequence (sequence No. 1).

3. Method of diagnosing haemachromatosis, **characterized in that** a biological sample is analyzed for the presence of nucleic acids which code for a TFR2 product with the amino acid sequence Pro Ala Val Ala Gin **Leu Ala Gly Gln** Leu Leu (Sequence No. 5) instead of the amino acid sequence Pro Ala Val Ala Gln **Ala Val Ala Gln** Leu Ala Gly Gln Leu Leu (Sequence No. 4) or for the presence of a TFR2-α gene product with an amino acid sequence Pro Ala Val Ala Gln **Leu Ala Gly Gln** Leu Leu (Sequence No. 5) instead of the amino acid sequence Pro Ala Val Ala Gin **Ala Val Ala Gln** Leu Ala Gly Gln Leu Leu (Sequence No. 4).

4. Method according to Claim 3, **characterized in that** a biological sample is analyzed for the presence of nucleic acids which code for a TFR2-α product with an amino acid sequence leucine-alanine-glycine-glutamine for the amino acids 594-597 or for the presence of TFR2-α gene product with an amino acid sequence leucine-alanine-glycine-glutamine for the amino acids 594-597.

5. Method according to one of the preceding claims, **characterized in that** the biological sample is also analyzed for the presence of nucleic acids whose TFR2 gene does not have the mentioned mutation or the mentioned altered amino acid sequence.

6. Method according to one of the preceding claims, **characterized in that** the analysis is carried out in a manner known per se by sequencing the nucleic acid obtained from the biological sample.

7. Method according to Claim 1, **characterized in that** nucleic acids from the biological sample are brought into contact with at least one probe having the capacity for hybridizing with a region of these nucleic acids which contains the nucleotide sequence 5' cccgccgtggcccagctcgcagggcagctcctc 3' (Sequence No. 3) and that a test is made whether corresponding hybridization products are present.

8. Method according to Claims 2 and 7, **characterized in that** nucleic acids from the biological sample are brought into contact with at least one probe which is capable of hybridizing with a region of these nucleic acids which corresponds to a region in Exon 16 of the TFR2-α cDNA sequence in the region of the nucleotides 1780-1791 when a deletion of the nucleotides 1780-1791 is present and **in that** it is tested whether corresponding hybridization products are present.

9. Method according to Claim 7, **characterized in that** nucleic acids of the biological sample are brought into contact with at least one probe which is capable of hybridizing with a region of these nucleic acids which contains the nucleotide sequence . 5' cccgccgtggcccaggccgtggcccagctcgcagggcagctcctc 3' (Sequence No. 2) and **in that** it is tested whether corresponding hybridization products are present.

10. Method according to Claim 8, **characterized in that** nucleic acids of the biological sample are brought into contact with at least one probe which is capable of hybridizing with a region of these nucleic acids which corresponds to a region in Exon 16 of the TFR2-α cDNA sequence in the region of the nucleotides 1780-1791 when there is no mutation present in the region of the nucleotides 1780-1791 and **in that** it is tested whether corresponding hybridization products are present.

11. Probe for the diagnosis of hemochromatosis, **characterized in that** the probe is capable of hybridization with nucleic acids of a biological sample in a region which contains the nucleotide sequence 5' cccgccgtggcccagctcgcagggcagctcctc 3' (Sequence No. 3) in Exon 16 of the TFR2 cDNA sequence.

12. Probe according to Claim 11, **characterized in that** the probe is capable of hybridization with nucleic acids of a biological sample in a region corresponding to a region in Exon 16 of TFR2-α cDNA sequence in the region of the nucleotides 1780-1791 when a deletion of the nucleotides 1780-1791 is present.

## Revendications

1. Procédé de diagnostic de l'hémochromatose, **caractérisé en ce que** l'on recherche sur un échantillon biologique la présence de la séquence de nucléotides 5' cccgccgtggcccagctcgcagggcagctcctc3' (séquence n°3) au lieu de la séquence de nucléotides 5' cccgccgtggcccaggccgtggcccagctcgcagggcagctcctc3' (séquence n°2) en raison d'une délétion d'une longueur de 12 nucléotides dans l'exon 16 de la séquence ADNc de TFR2.

2. Procédé suivant la revendication 1, **caractérisé en ce que** l'on recherche sur un échantillon biologique la présence d'une délétion des nucléotides 1780 à 1791 dans l'exon 16 de la séquence ADNc de TFR2-α (séquence n°1).

3. Procédé de diagnostic de l'hémochromatose, **caractérisé en ce que** l'on recherche sur un échantillon biologique la présence d'acides nucléiques qui codent pour un produit de TFR2 et qui ont une séquence d'acides aminés ProAlaValAlaGln**LeuAlaGlyGln**LeuLeu (séquence n°5) au lieu de la séquence d'acides aminés ProAlaValAlaGln**AlaValAlaGln**LeuAlaGlyGlnLeuLeu (séquence n°4) ou la présence d'un produit de gène de TFR2-α ayant une sequence d'acides aminés ProAlaValAlaGln**LeuAlaGlyGln**LeuLeu (sequence n°5) au lieu de la séquence d'acides aminés ProAlaValAlaGln**AlaValAlaGln**LeuAlaGlyGlnLeuLeu (sequence n°4).

4. Procédé suivant la revendication 3, **caractérisé en ce que** l'on recherche sur un échantillon biologique la présence d'acides nucléiques qui codent pour un produit de TFR2-α et qui ont une sequence d'acides amines leucine-alanine-glycine-glutamine pour les acides aminés 594 à 597 ou la présence d'un produit de gène de TFR2-α ayant une séquence d'acides aminés leucine-alanine-glycine-glutamine pour les acides aminés 594 à 597.

5. Procédé suivant l'une des revendications précédentes, **caractérisé en ce que** l'on recherche sur l'échantillon biologique également la présence d'acides nucléiques dont le gène de TFR2 n'a pas ladite mutation ou ladite séquence modifiée d'acides aminés.

6. Procédé suivant l'une des revendications précédentes, **caractérisé en ce qu'**on effectue la recherche d'une manière en soi connue par séquençage des acides nucléiques obtenus à partir de l'échantillon biologique.

7. Procédé suivant la revendication 1, **caractérisé en ce que** l'on met des acides nucléiques de l'échantillon biologique en contact avec au moins une sonde qui est apte à s'hybrider avec les acides nucléiques qui contient la séquence de nucléotides 5'cccgccgtggcccagctcgcagggcagctcctc3' (sequence n°3) et **en ce que** l'on contrôle s'il y a des produits d'hybridation correspondants.

8. Procédé suivant la revendication 2 et 7, **caractérisé en ce que** l'on met des acides nucléiques de l'échantillon biologique en contact avec au moins une sonde qui est apte à s'hybrider avec une region de ces acides nucléiques qui correspond à une région de l'exon 16 de la séquence d'ADNc de TFR2-α dans la région des nucléotides 1780 à 1791 lorsqu'il y a une délétion des nucléotides 1780 à 1791 et **en ce que** l'on contrôle s'il y a des produits d'hybridation correspondants.

9. Procédé suivant la revendication 7, **caractérisé en ce que** l'on met des acides nucléiques de l'échantillon biologique en contact avec au moins une sonde qui est apte à s'hybrider avec une region de ces acides nucléiques qui contient la sequence de nucleotides 5'cccgccgtggcccaggccgtggcccagctcgcagggcagctcctc3' (sequence n°2) et **en ce que** l'on contrôle s'il y a des produits d'hybridation correspondants.

10. Procédé suivant la revendication 8, **caractérisé en ce que** l'on met des acides nucléiques de l'échantillon biologique en contact avec au moins une sonde qui est apte à s'hybrider avec une région de ces acides nucléiques qui correspond à une région de l'exon 16 de la séquence d'ADNc de TFR2-α dans la région des nucléotides 1780 à 1791 s'il n'y a pas de mutation dans la région des nucléotides 1780 à 1791 et **en ce que** l'on contrôle s'il y a des produits d'hybridation correspondants.

11. Sonde de diagnostic de l'hémochromatose, **caractérisée en ce que** la sonde est apte à s'hybrider avec des acides nucléiques d'un échantillon biologique dans une région qui contient la séquence de nucléotides 5'cccgccgtggcccagctcgcagggcagctcctc3' (séquence n°3) de l'exon 16 de la séquence d'ADNc de TFR2.

12. Sonde suivant la revendication 11, **caractérisée en ce que** la sonde est apte à s'hybrider à des acides nucléiques d'un échantillon biologique dans une région qui correspond à une région de l'exon 16 de la séquence d'ADNc de TFR2-α dans la région des nucléotides 1780 à 1791 s'il y a une délétion des nucléotides 1780 à 1791.
